# EUROPEAN PATENT APPLICATION

(11) **EP 3 342 397 A1**
(43) Date of publication of application: **04.07.2018**
(21) Application number: 17020600.7
(22) Date of filing: 31.12.2017
(51) Int. Cl.: A61K 9/00, A61K 31/00

(54) **A PHARMACEUTICAL SPRAY DOSAGE FORM COMPRISING NAPROXEN AND LIDOCAINE FREE BASE**

(30) Priority: 31.12.2016 TR 201620500
(71) Applicant: Abdi Ibrahim Ilac Sanayi ve Ticaret A.S., 34500 Esenyurt/Istanbul (TR)
(72) Inventor: Abdi Ibrahim Ilac Sanayi ve Ticaret A.S., 34500 Esenyurt/Istanbul (TR)

(57) **Abstract**

Present invention relates to a spray formulations for topical administration comprising analgesic and anesthesic agents. Particularly, present invention relates to formulations in solution and spray forms comprising naproxen and lidocain and said formulations are free of preservatives.

## Description

Present invention relates to a spray formulations for topical administration comprising analgesic and anesthesic agents. Particularly, present invention relates to formulations in solution and spray forms comprising naproxen and lidocain and said formulations are free of preservatives.

### Technical Field of Invention

Naproxen is an analgesic, ant-inflammatory, antipyretic drug that is a member of non-steroidal anti-inflammatory drugs (NSAIDs) group. It was first described in the patent US3904682. Chemical name is (+)-2-(6-Methoxy-2-naphthyl) propionic acid and its chemical structure is as shown in formula I.

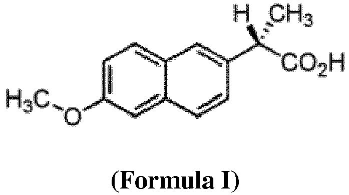

Naproxen is white or off-white, almost odorless, crystalline powder. Practically insoluble in water, soluble in alcohol, chloroform, and methyl alcohol, and slightly soluble in ether.

When it is topically applied to skin in gel form, 30% of active substance is easily absorbed.

Lidocaine is a local anesthetic substance. There are ointment, gel, spray or oral solution preparations to be used for local anesthetic effect and injectable preparations for use as local anesthetic. Lidocaine was first described in the patent US2441498. Chemical name is 2-(diethylamino)-N-(2,6-dimethylphenyl)acetamide and its chemical structure is as shown in formula II.

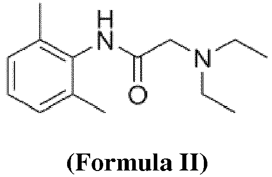

While hydrochloride salt of lidocaine is soluble in water, free base form is practically insoluble in water. It is soluble in alcohol and chloroform and freely soluble in benzene and ether.

KR20070059758 relates to transdermal application of non-steroidal antiinflammatory drugs (ibuprofen, ketoprofen, flurbiprofen, fenoprofen, naproxen, piroxicam, tenoxicam, isoxicam, meloxicam, indomethacin, aceclofenac or diclofenac) and local anesthetics (procaine, chlorcaine, tetracaine, prilocaine, lidocaine, mepivacaine, bupivacaine, ropivacaine or etidocaine). Although, many active substances were mentioned as non-steroidal anti-inflammatory drugs and local anesthetics in this patent document, the combination of naproxen and lidocaine was not mentioned specifically.

Dogan E et al. studied anesthetic effect of intrauterine lidocaine and oral naproxen sodium combination in endometrial biopsy (Obstet Gynecol. 2004 February; 103(2):347-51). In this study, naproxen sodium was given by oral route and local administration was not mentioned.

Spray products comprising mentioned active substances or combinations thereof, does not exist in the market. Inventors surprisingly developed a spray formulation comprising both active agents and developed formulation is the first in the world.

Vemcaine Pump spray® is a formulation in a spray form comprising only lidocaine as active agent and ethanol (96%), PEG, banana aroma, mint, sodium saccharide and water as excipients. Said formulation comprises only lidocaine active agent and is a preparation administered to only mucosa wherein it does not comprise naproxen.

Due to remaining of gel formulations on skin, which is one of the biggest problems, some patients may give up their treatment voluntarily. Especially for the gel formulations containing active agent more than 5%, white residuals can occur after applied to the skin. Prevention of the white residuals after application is important to provide patients to continue their treatment. Although gel has a tendency to form a layer on the skin due to its polymeric structure, it takes time for skin to absorb active agent embedded in the gel matrix. Since absorption of spray formulations are more rapid than the absorption of gel formulations by skin, spray formulations are preferred when acute effect is desired.

In the present invention, absorption of skin is accelerated by penetration enhancer excipients like ethanol and propylene glycol. Polyethylene glycol (PEG) is used as film forming agent in order to provide formulation to hold on the skin.

Like lidocaine free base form, naproxen active substance is also practically insoluble in water. Therefore, it is very difficult to formulate these two water insoluble active substances in a stable spray form.

Moreover, because naproxen and lidocaine are stable at neutral pH and basic pH, respectively, to formulate both active substances in a single dosage form poses a risk with respect to stability.

Considering the above mentioned prior arts, the objective of the present invention is to provide a formulation that comprises naproxen and lidocaine free base in a stable spray form.

### Detailed Description of the Invention

The inventors surprisingly succeeded to formulate naproxen and lidocaine free base in a stable single spray dosage form.

In this respect, present invention is related to a spray formulations comprises naproxen and lidocaine free base.

In another embodiment of the present invention, inventors achieved to formulate a stable single spray dosage form comprising both active agents which are stable at different pH values. The inventors obtained a stable solution by providing an optimum dissolution medium for both active substances. A spray formulation produced by excipients used in the present invention forms a thin layer on the skin after administration. Then it is absorbed by skin rapidly. Present invention comprising both two active agents do not leave residuals after application to the skin.

In a present invention, pH regulating agent which is necessary to obtain a stable spray solution comprising both two active agents can be selected among triethanolamine (TEA), amine base tromethamine, tetrahydroxypropyl ethylenediamine, diethanolamine, aminomethyl propanol, and/or sodium or ammonium hydroxide at a concentration less than 20% w/w or mixtures thereof and is not limited to thereto.

In a formulation of a present invention, preferably triethanolamine (TEA) was used as pH regulating agent. Triethanolamine is in the range of 5-35% of the total weight of formulation, preferably in the range of 4-12%.

In a present invention, solvent which is necessary to obtain a stable spray solution can be selected among water, ethanol, isopropyl alcohol, propylene glycol, polyethylene glycol, benzyl alcohol, diethyl ether and dimethoxy and/or mixtures thereof and is not limited thereto. In the present invention water, ethanol, propylene glycol and/or mixtures thereof were used as solvent.

In the present invention, ethanol is in the range of 5-60% of the total weight of formulation, preferably in the range of 25-50%.

In the present invention, propylene glycol is in the range of 5-40% of the total weight of formulation.

In another embodiment of the present invention, PEG 400, PEG or derivatives at different molecule weight, propylene glycol and/or mixtures thereof were used.

In the present invention, PEG is in the range of 1-20% of the total weight of formulation, preferably in the range of 3-12%.

In another embodiment of the present invention, by using water, ethanol and propylene glycol together as solvent in the formulation, triethanolamine amount in the formula was decreased by 50-60%, antiseptic property was gained to the finished product, a good dissolution medium was provided for the active agents.

In another embodiment of the present invention, type and amount of pharmaceutically acceptable excipients are selected carefully that antimicrobial preservative property of the present invention is gained without the addition of antimicrobial preservatives such as as parabens, benzoic acid, sorbic acid. As stated in the literature, in formulations where alcohol concentration is more than 15% and propylene glycol concentration is between 15% and 30%, the antimicrobial activity can be provided without using an antimicrobial preservative. Particularly, since spray formulations have a rheology of low viscosity and a high content of water, they have a tendency to degradation and microbial growth.

In the present invention, the amount of ethanol and propylene glycol in the formulation are selected specifically to provide both microbial protection free from antimicrobial preservatives and to solve the active ingredients.

In the present invention, propylene glycol not only forms a dissolution medium for lidocaine base to solve but also increases the penetration of spray formulation to the skin. It also acts as a disinfectant when used in combination with ethanol in the formulation free of preservatives.

In the present invention, pH range that both active agents are stable in the formulation is between 6.5 and 8.5, preferably in the range of 7.0-7.5.

In the present invention, formulation comprises naproxen in the range of 5-20% of the total weight of formulation. In other words, the amount of naproxen in the finished product is 5-20% of the total formulation.

In the present invention, formulation comprises lidocaine in the range of 1-10% of the total weight of formulation. In other words, the amount of lidocaine in the finished product is 1-10% of the total formulation.

In a present invention, antioxidant can be selected among sodium metabisulphite, potassium metasulphite, buthyl hydroxyl toluene(BHT), alpha tocopherol, ascorbic acid, ascorbic palmitate, sodium sulphite, sodium thiosulfate and/or mixtures thereof and is not limited thereto. In the present invention sodium metabisulphite was used as an antioxidant. In the present invention, the amount of sodium metabisulphite is in the range of 0.05%- 5% by weight of the total formulation weight, preferably it is in the range of about 0.05%- 1%.

The spray formulation of the invention offers many advantages such as easy spreading and rapid absorption, no residue on the skin, stability during the shelf life. Besides, said spray formulation does not contain parabens that cause skin irritation and contact dermatitis.

Unless otherwise stated, all weights and ratios mentioned in this description are expressed in terms of weights and all weight percentages are provided in proportion to the weight of the total formulation.

The present invention will be described more in details by way of the following example. The example does not restrict the scope of the invention and should be considered in the light of the details of the above-given description.

### Example 1:

| **Raw materials** | **Amount (g)** |
|---|---|
| Naproxen | 10.0 |
| Lidocaine Base | 5.0 |
| Sodium Metabisulfite | 0.1 |
| Ethanol | 25.0 |
| Triethanolamine | 8.9 |
| Deionized Water | 51.0 |

### Process Steps:

1. Add ethanol in a container and mix.
2. Add sodium metabisulphite to the mixture obtained in 1 and mix until dissolved.
3. Add Lidocaine Base to the mixture obtained in 1 and mix until dissolved.
4. Add deionized water and triethanolamine to the mixture obtained in 1 and mix until dissolved and mix.
5. Add Naproxen to the mixture described in the step no.1 and mix until dissolved.

### Result:

As a result of trial, a clear spray solution free from any undissolved particles was obtained. However, there is a need for improvement since it does not contain any excipient required for spray applications and capable of forming a film. Since water and ethanol mixture can not form enough film in the applied region, it can easily flow through the skin surface during application.

### Example 2:

| **Raw Materials** | **Amount (g)** |
|---|---|
| Naproxen | 10.0 |
| Lidocaine Base | 5.0 |
| Sodium Metabisulfite | 0.1 |
| Ethanol | 25.0 |
| Triethanolamine | 8.9 |
| Propylene Glycol | 51.0 |

1. Add ethanol and propylene glycol to a container and mix.
2. Add sodium metabisulfite to the mixture obtained in 1 and mix until dissolved.
3. Add lidocaine base to the mixture obtained in 1 and mix until dissolved.
4. Add triethanolamine to the mixture obtained in 1 and mix.
5. Add naproxen to the mixture obtained in 1 and mix until dissolved.

### Result:

As a result of trial, it is observed that formulation precipitates and agglomerates after addition of naproxen. In systems containing water and ethanol, when ethanol amount is less than 3 times the amount of naproxen, the water/TEA ratio required to dissolve 1 g of Naproxen should be between 0.2/0.7 and 6.0/3.5.

### Example 3:

| **Raw Materials** | **Amount (g)** |
|---|---|
| Naproxen | 10.0 |
| Lidocaine Base | 5.0 |
| Sodium Metabisulfite | 0.1 |
| Ethanol | 35.0 |
| Propylene Glycol | 20.0 |
| PEG 400 | 5.0 |
| Triethanolamine | 6.0 |
| Deionized Water | 18.9 |

### Process Steps:

1. Add ethanol and propylene glycol to a container and mix.
2. Add sodium metabisulfite to the mixture obtained in 1 and mix until dissolved.
3. Add lidocaine base to the mixture obtained in 1 and mix until dissolved.
4. Add deionized water and triethanolamine to the mixture obtained in 1 and mix.
5. Add PEG 400 to the mixture obtained in 1 and mix.
6. Add naproxen to the mixture obtained in 1 and mix until dissolved.

### Result:

As a result of trials, a clear spray solution was obtained. Besides, a film that is easily spreadable and penetrable to the skin was obtained by the addition of PEG 400 and propylene glycol to the formulation.

Inventors observed that the invention was stable in the stability studies conducted under 25° C /60% RH (Relative Humidity), 30°C / 65% RH and 40°C / 75% RH. Table 1 and Table 2 relates to the stability studies of the formulation described in Example 3.

**Table 1 - Naproxen/Lidocaine Spray Impurity Stability Study**

| | **Initial** | **1^{st} Month 40°C / 75% RH** | **3^{rd} Month 25 °C / 60% RH** | **3^{rd} Month 30°C / 65% RH** | **3^{rd} Month 40°C / 75% RH** |
|---|---|---|---|---|---|
| Lido Impurity A | < LOQ | 0.101 | 0.06 | 0.08 | 0.132 |
| Lido Impurity B | < LOQ | < LOQ | < LOQ | < LOQ | < LOQ |
| Max. Unknown Impurity | 0.086 (RRT 6.07) | 0.218 (RRT 0.58) | 0.136 (RRT 2.03) | 0.182 (RRT 2.03) | 0.487 (RRT 5.40) |
| Total Impurities | 0.182 | 0.483 | 0.258 | 0.535 | 1.152 |
| *LOQ: Limit of quantification* | | | | | |
| *ND: Not Detected.* | | | | | |
| *RRT: Relative retention time* | | | | | |

**Table 2 - Naproxen/Lidocaine Spray Assay Stability Study**

| | **Initial** | **1^{st} Month 40 °C / 75% RH** | **3^{rd} Month 25 °C / 60% RH** | **3^{rd} Month 30 °C / 65% RH** | **3^{rd} Month 40 °C / 75% RH** |
|---|---|---|---|---|---|
| Lidocaine (%) | 100.2 | 98.9 | 100.7 | 99.3 | 100.2 |
| Naproxen (%) | 100.7 | 101.2 | 102.9 | 101.2 | 101.3 |

## Claims

1. A pharmaceutical composition **characterized by** a spray dosage form comprising naproxen and lidocaine free base.

2. A spray formulation according to claim 1, wherein the amount of naproxen is in the range of 5-20% of the total weight of the formulation.

3. A spray formulation according to claim 1, wherein the amount of lidocaine free base is in the range of 1-10% of the total weight of the formulation.

4. A spray formulation according to claim 1, wherein it comprises pH regulating agent-solvent system.

5. A spray formulation according to claim 4, wherein pH regulating agent is selected among triethanolamine (TEA), amine base tromethamine, tetrahydroxypropyl ethylenediamine, diethanolamine, aminomethyl propanol, and/or sodium or ammonium hydroxide at a concentration less than 20% w/w ror mixtures thereof.

6. A spray formulation according to claim 5, pH regulating agent is triethanolamine (TEA).

7. A spray formulation according to claim 6, wherein triethanolamine is in the range of 5-35% of the total weight of formulation.

8. A spray formulation according to claim 7, wherein triethanolamine is in the range of 4-12%.

9. A spray formulation according to claim 4, wherein solvent is selected among water, ethanol, isopropyl alcohol, propylene glycol, polyethylene glycol, benzyl alcohol, diethyl ether and dimethoxy and/or mixtures thereof.

10. A spray formulation according to claim 9, wherein solvent is water, ethanol, propylene glycol and/or mixtures thereof.

11. A spray formulation according to claim 9 and 10, wherein ethanol is in the range of 5-60% of the total weight of formulation.

12. A spray formulation according to claim 9 and 10, ethanol is in the range of 25-50%.

13. A spray formulation according to claim 9 and 10, propylene glycol is in the range of 5-40% of the total weight of formulation.

14. A spray formulation according to claim 1, wherein the pH of the formulation is between 6.5 and 8.5.

15. A spray formulation according to claim 1, wherein the pH of the formulation is in the range of 7.0-7.5.

16. A spray formulation according to claim 1, wherein the formulation is free of preservatives.

17. A spray formulation according to claim 1, wherein the formulation comprises antioxidant.

18. A spray formulation according to claim 17, wherein antioxidant is selected among sodium metabisulphite, potassium metasulphite, buthyl hydroxyl toluene(BHT), alpha tocopherol, ascorbic acid, ascorbic palmitate, sodium sulphite, sodium thiosulfate and/or mixtures thereof
